Europäisches Patentamt

European Patent Office

Office européen des brevets

(11) Numéro de publication : **0 455 532 A1**

(19)

(12) # DEMANDE DE BREVET EUROPEEN

(21) Numéro de dépôt : **91401019.4**

(51) Int. Cl.⁵ : **C09K 5/00, B01D 53/34**

(22) Date de dépôt : **17.04.91**

(30) Priorité : 03.05.90 FR 9005579

(43) Date de publication de la demande :
06.11.91 Bulletin 91/45

(84) Etats contractants désignés :
AT BE CH DE DK ES FR GB GR IT LI LU NL SE

(71) Demandeur : ATOCHEM
4, Cours Michelet La Défense 10 Cédex 42
F-92091 Paris La Défense (FR)

(72) Inventeur : Cauvy, Daniel
43, rue Saint Hubert
F-57220 Bambiderstroff (FR)
Inventeur : Couget, Francis
31, rue Croix
F-57350 Striring-Wendel (FR)
Inventeur : Slota, Raymond
2, rue de Vivonne
F-57890 Porcelette (FR)
Inventeur : Villemin, Bernard
2, rue Le Chatelier
F-60550 Verneuil en Halatte (FR)

(54) **Procédé de refroidissement associé à un procédé utilisant de l'acide fluorhydrique anhydre.**

(57) Ce procédé est destiné à évacuer au moins une partie de la chaleur produite dans au moins une réaction et/ou opération de purification mettant en jeu de l'HF anhydre, le refroidissement étant effectué par échange thermique avec un fluide de refroidissement circulant dans un circuit qui comporte au moins une zone d'échange (9 ; 10 ; 11) présentant un risque de fuite de l'HF dans le fluide de refroidissement. On utilise, comme tel fluide, une solution aqueuse d'au moins un composé choisi parmi (a) les bases fortes ; (b) les sels résultant de la combinaison d'une base forte et d'un acide organique ou minéral dont le pKA est >3,5 ; (c) les sels d'oxydes amphotères avec les bases ; (d) les composés qui, au contact de l'eau, se transforment en l'un des composés précités ; on fait circuler ledit fluide de refroidissement en circuit fermé ; et on travaille avec une pression de fluide de refroidissement inférieure à la pression à laquelle on conduit la réaction et/ou l'opération de purification.

FIG. 2

EP 0 455 532 A1

Jouve, 18, rue Saint-Denis, 75001 PARIS

La présente invention porte sur un procédé de refroidissement destiné à évacuer au moins une partie de la chaleur produite dans au moins une réaction et/ou au moins une opération de purification mettant en jeu de l'acide fluorhydrique anhydre ou substantiellement anhydre, ledit refroidissement étant effectué par échange thermique avec un fluide de refroidissement maintenu à une température désirée et circulant dans un circuit qui comporte au moins une zone d'échange présentant un risque de fuite de l'acide fluorhydrique dans le fluide de refroidissement.

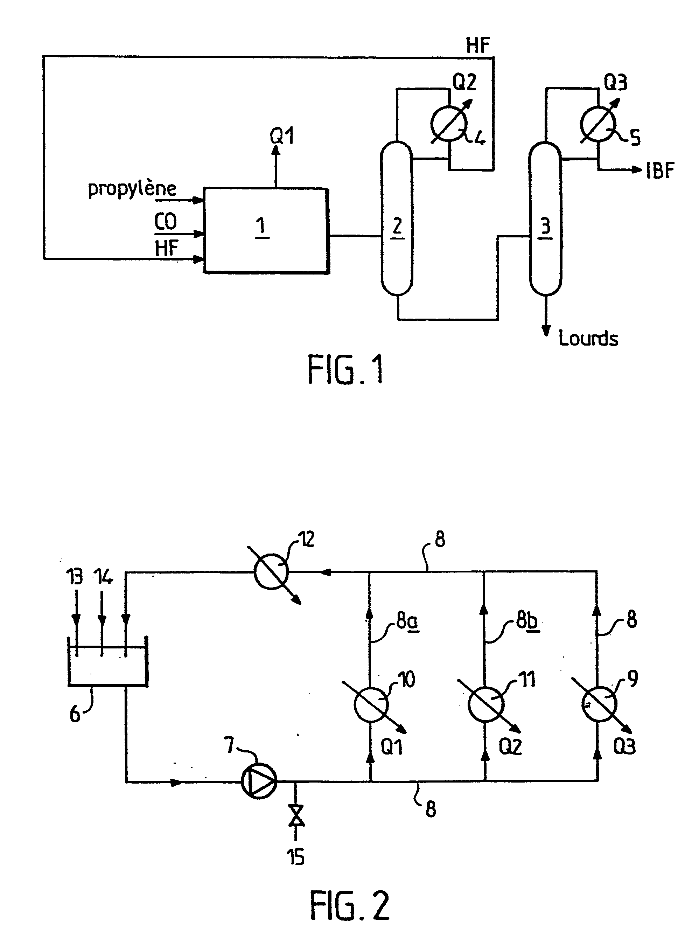

A titre d'exemple, on peut mentionner la réaction de carbonylation d'un alcène par l'oxyde de carbone et l'acide fluorhydrique anhydre ou substantiellement anhydre, en particulier la fabrication de fluorure d'isobutyryle à partir de la réaction exothermique, conduite sous une pression de l'ordre de 10200 bars, de propylène, d'oxyde de carbone et d'acide fluorhydrique, l'acide fluorhydrique étant introduit en excès dans la réaction. L'appareillage pour la mise en oeuvre de ce procédé est représenté de façon schématique sur la Figure 1 du dessin annexé. La réaction proprement dite est conduite dans le réacteur 1, la chaleur de réaction Q1 devant être éliminée par un circuit de refroidissement. Le fluorure d'isobutyryle brut est adressé à une première colonne de distillation 2 pour séparer, en tête, l'acide fluorhydrique que l'on recycle, puis à une deuxième colonne de distillation 3, permettant de séparer, en tête, le fluorure d'isobutyryle, et, en pied de colonne, les lourds. A chacune de ces colonnes 3, 4 est associé un condenseur respectivement 4, 5, d'où des quantités de chaleur respectivement Q2 et Q3 à éliminer vers le circuit de refroidissement.

Dans un circuit de refroidissement classique, utilisant de l'eau comme fluide de refroidissement, toute fuite d'acide fluorhydrique dans l'eau crée localement, ou sur l'ensemble du réseau, une situation de haute corrosion, risquant de mettre en danger la vie de l'installation, avant que l'on ait pu remédier aux fuites. Même si l'on réagit vite et que l'on vidange le circuit, de l'acide fluorhydrique dilué peut se trouver emprisonné dans des zones mal purgeables.

On peut envisager d'utiliser, comme fluide de refroidissement, à la place de l'eau, un fluide non-aqueux, tel que de l'huile ou un Fréon. Le dimensionnement des échangeurs aboutit alors à des coûts de fonctionnement très élevés. Par ailleurs, en cas de fuite, on est amené à vidanger un tel fluide non-aqueux contenant de l'acide fluorhydrique et il n'existe pas de solution simple permettant de réutiliser un tel fluide une fois qu'il a été pollué.

La présente invention vise à remédier à ces inconvénients et propose d'utiliser, pour le refroidissement, un circuit fermé d'eau, éventuellement déminéralisée, ayant subi un traitement basique préalable. En cas de fuite, locale ou plus importante, l'acide fluorhydrique se trouvera très rapidement sous la forme d'un ou de fluorures beaucoup moins agressifs pour les matériaux que l'acide fluorhydrique. Le circuit, lorsque sa teneur en fluorures sera significative, devra bien sûr être purgé ; cependant, après précipitation des fluorures à la chaux par exemple, l'eau pourra être rejetée sans dommage pour l'environnement. Le circuit fermé d'eau ainsi traitée sera maintenu à la température adéquate par toute technologie connue (échangeur refroidi par toutes sources de frigories, y compris aéroréfrigérants).

Le procédé de refroidissement selon l'invention, tel qu'il a été défini dans l'introduction de la présente description, est donc caractérisé par le fait :

– qu'on utilise, comme fluide de refroidissement, une solution aqueuse d'au moins un composé dont la concentration peut aller jusqu'à la limite de saturation et qui est choisi parmi :

(a) les bases fortes ;

(b) les sels résultant de la combinaison d'une base forte et d'un acide organique ou minéral dont le pKA est supérieur à 3,5 ;

(c) les sels d'oxydes amphotères avec les bases ; et

(d) les composés qui, au contact de l'eau, se transforment en l'un des composés précités ;

– qu'on fait circuler ledit fluide de refroidissement en circuit fermé ; et

– qu'on travaille avec une pression de fluide de refroidissement inférieure à la pression à laquelle on conduit la réaction et/ou l'opération de purification.

Comme base forte (a), on peut citer les hydroxydes de métaux alcalins ou alcalino-terreux, tels que les hydroxydes de sodium, de potassium et de calcium, et les hydroxydes d'ammonium quaternaire aliphatiques.

Comme sels (b), on peut mentionner les sels d'une base forte, tels que ceux indiqués au paragraphe précédent, et d'un acide organique ou minéral dont le pKA est supérieur à 3,5 et, de préférence, supérieur à 7, comme les acides acétique, carbonique, silicique et phosphorique. On peut ainsi mentionner le silicate de sodium, le phosphate de sodium et le carbonate de sodium.

Comme sels (c), on peut citer les aluminates de sodium ou de potassium.

Comme composé (d), on peut citer les alcoolates de métaux alcalins ou alcalino-terreux.

Le fluide de refroidissement étant toujours à une pression inférieure à la pression du circuit dans lequel se déroule le procédé, ce dernier est donc intrinsèquement protégé en cas de fuite. Par ailleurs, le circuit de refroidissement est, quant à lui, protégé de la corrosion par le choix du fluide de refroidissement selon l'invention.

Conformément à une caractéristique avantageuse du procédé selon l'invention, on vidange le circuit de refroidissement lorsque sa teneur en fluorure atteint la valeur susceptible de provoquer une corrosion dans le circuit, et on provoque la précipitation des fluorures avant de rejeter l'eau sans dommage pour l'environnement.

Sur la Figure 2 du dessin annexé, on a représenté, de façon schématique, le circuit de refroidissement associé au circuit de procédé de la Figure 1.

Le fluide de refroidissement de ce circuit est contenu dans une cuve 6 et il est mis en circulation grâce à une pompe de recirculation 7 à travers l'installation. Il chemine dans une canalisation 8 en passant dans un échangeur de chaleur 9 avant d'être renvoyé dans la cuve 6. Le circuit comporte encore deux branches 8a et 8b disposées de façon parallèle, en réunissant des points situés en amont de l'échangeur 9 à des points situés en aval de celui-ci ; de la même façon, chaque branche 8a, 8b comporte une zone d'échange respectivement 10 et 11. Les échangeurs 9, 10 et 11 sont ceux présentant le risque de fuite d'acide fluorhydrique, devant éliminer les quantités de chaleur respectivement Q3, Q1 et Q2 définies ci-dessus en relation avec la Figure 1. Un échangeur servant à refroidir le circuit primaire qui vient d'être décrit par tous moyens connus de l'homme du métier est représenté en 12, juste en amont de la cuve 6. On a par ailleurs symbolisé en 13 et 14 l'introduction éventuelle d'un appoint d'eau et l'introduction de l'agent selon l'invention de traitement de l'eau. On peut par ailleurs effectuer des prélèvements pour analyse en 15.

Pour simuler les effets d'une fuite d'acide fluorhydrique vers un circuit de refroidissement pour un procédé utilisant de l'acide fluorhydrique anhydre, on a réalisé un montage de laboratoire analogue à celui représenté sur la Figure 2, à ceci près qu'il ne comporte pas les échangeurs 9, 10 et 11. Tous les éléments de ce montage sont en polypropylène. L'échangeur 12 sert, dans ce cas, à maintenir le fluide du circuit à température constante par circulation d'eau thermostatée. Par ailleurs, dans la cuve 6, on dispose des éprouvettes en acier au carbone et en acier inox AISI 304 destinées à la mesure de la vitesse de corrosion dans les différents cas. A la fin de chacune des expériences relatées dans les Exemples et Exemples comparatifs suivants, on mesure l'épaisseur de la couche de corrosion.

## Exemple 1

On place dans le circuit 2260 g d'eau déminéralisée. On met la pompe en service et on introduit 120 g (3 moles) de soude pure (NaOH) en pastilles. En l'espace d'une heure, on ajoute 50 g d'une solution aqueuse à 70% d'acide fluorhydrique (1,75 mole). Après 48 heures à 40°C, on prélève les éprouvettes et l'on constate que ni l'acier au carbone ni l'acier inox AISI 304 ne montrent de traces de corrosion.

## Exemple 2 (Comparatif)

On procède comme à l'Exemple 1, mais en l'absence de l'ajout préalable de soude. On note une corrosion des éprouvettes de 20 mm/an pour l'acier au carbone et 7 mm/an pour l'acier inox AISI 304.

## Exemple 3

On utilise le montage de l'Exemple 1, et on place, dans le circuit, 2600 g d'eau déminéralisée, et 690 g de carbonate de potassium $K_2CO_3$ (5 moles). En l'espace d'une heure, on ajoute 100 g d'une solution aqueuse à 708 d'acide fluorhydrique (3,5 moles). On maintient la température à 40°C pendant 48 heures. La corrosion des éprouvettes en acier au carbone et en acier inox AISI 304 est si minime qu'elle ne peut être mesurée.

## Exemple 4 (Comparatif)

On procède comme à l'Exemple 3, mais sans addition de carbonate de potassium. On relève une corrosion de 37 mm/an et de 12 mm/an respectivement pour l'acier au carbone et pour l'acier AISI 304.

## Revendications

1 - Procédé de refroidissement destiné à évacuer au moins une partie de la chaleur produite dans au moins une réaction et/ou au moins une opération de purification mettant en jeu de l'acide fluorhydrique anhydre ou substantiellement anhydre, ledit refroidissement étant effectué par échange thermique avec un fluide de refroidissement maintenu à une température désirée et circulant dans un circuit qui comporte au moins une zone d'échange (9 ; 10 ; 11) présentant un risque de fuite de l'acide fluorhydrique dans le fluide de refroidissement, caractérisé par le fait :

    – qu'on utilise, comme fluide de refroidissement, une solution aqueuse d'au moins un composé dont la concentration peut aller jusqu'à la limite de saturation et qui est choisi parmi :

        (a) les bases fortes ;

        (b) les sels résultant de la combinaison d'une base forte et d'un acide organique ou minéral dont le pKA est supérieur à 3,5 ;

        (c) les sels d'oxydes amphotères avec les bases ; et

        (d) les composés qui, au contact de l'eau, se transforment en l'un des composés précités ;

    – qu'on fait circuler ledit fluide de refroidissement en circuit fermé ; et

    – qu'on travaille avec une pression de fluide de refroidissement inférieure à la pression à laquelle on conduit la réaction et/ou l'opération de purification.

**2** - Procédé selon la revendication 1, caractérisé par le fait qu'on utilise, comme base forte (a), un hydroxyde de métal alcalin ou alcalino-terreux ou un hydroxyde d'ammonium quaternaire aliphatique.

**3** - Procédé selon la revendication 1, caractérisé par le fait qu'on utilise, comme sel (b), un sel d'une base forte et d'un acide choisi parmi les acides acétique, carbonique, silicique ou phosphorique.

**4** - Procédé selon la revendication 1, caractérisé par le fait qu'on utilise, comme sel (c), un aluminate de sodium ou de potassium.

**5** - Procédé selon la revendication 1, caractérisé par le fait qu'on utilise un alcoolate de métal alcalin ou alcalino-terreux comme composé (d).

**6** - Procédé selon l'une des revendications 1 à 5, caractérisé par le fait qu'on vidange le circuit de refroidissement lorsque sa teneur en fluorure atteint la valeur susceptible de provoquer une corrosion dans le circuit, et qu'on provoque la précipitation des fluorures avant de rejeter l'eau sans dommage pour l'environnement.

**7** - Procédé selon l'une des revendications 1 à 6, appliqué au circuit de refroidissement associé à la réaction de carbonylation d'un alcène par l'oxyde de carbone et l'acide fluorhydrique.

FIG.1

FIG.2

Office européen
des brevets

**RAPPORT DE RECHERCHE EUROPEENNE**

Numero de la demande

EP 91 40 1019

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | US-A-3 811 246 (ERICKSON)<br>* Revendications 1,2,4; figure 2; colonne 5, lignes 54-66 *<br>--- | 1,6,7 | C 09 K 5/00<br>B 01 D 53/34 |
| A | US-A-2 983 449 (DREYER)<br>* Revendications 1,3; figure 3 *<br>--- | 1,6,7 | |
| A | FR-A-2 606 772 (SOCIETE CHIMIQUE DES CHARBONNAGES)<br>* Revendications 1,8 *<br>--- | 1 | |
| A | FR-A-2 607 132 (SOCIETE CHIMIQUE DES CHARBONNAGES)<br>----- | | |

**DOMAINES TECHNIQUES RECHERCHES (Int. Cl.5)**

C 09 K
B 01 D

Le présent rapport a été établi pour toutes les revendications

| Lieu de la recherche | Date d'achèvement de la recherche | Examinateur |
|---|---|---|
| LA HAYE | 01-08-1991 | NICOLAS H.J.F. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)